# EUROPÄISCHE PATENTANMELDUNG

(11) **EP 1 702 681 A2**
(43) Veröffentlichungstag der Anmeldung: **20.09.2006**
(21) Anmeldenummer: 06004175.3
(22) Anmeldetag: 02.03.2006
(51) Int. Cl.: B01J 25/00, B01J 25/02, B01J 35/04, B01J 37/02, C07C 5/02, C07C 1/04, C07C 1/12, C11C 3/12

(54) **Katalysatorformkörper, insbesondere zur Verwendung als Katalysatoren bei der Hydrierung**

(30) Priorität: 08.03.2005 DE 102005011047
(71) Anmelder: H.C. Starck GmbH, 38642 Goslar (DE)
(72) Erfinder: Turek, Thomas, Dr., 38640 Goslar (DE); Wolf, Aurel, Dr., 45279 Essen (DE); Münnich, Christian, Dr., 51061 Köln (DE); Meisel, Rainer-Leo, Dr., 79725 Laufenburg (DE); Zimmermann, Stefan, Dr., 79725 Laufenburg (DE)
(74) Vertreter: Perchenek, Nils

(57) **Zusammenfassung**

Die vorliegende Erfindung betrifft einen Katalysatorformkörper, insbesondere zur Verwendung als Katalysatoren bei der Hydrierung.

## Beschreibung

Die vorliegende Erfindung betrifft einen Katalysatorformkörper, insbesondere zur Verwendung als Katalysatoren bei der Hydrierung.

Heterogene Hydrierkatalysatoren auf Basis von Nickel und anderer Elemente oder gegebenenfalls weiterer für die Hydrierung geeigneter Elemente, wie Co oder Cu und mindestens einem weiteren katalytisch unwirksamen Metall, wie insbesondere Aluminium, finden Anwendung in der Hydrierung von organischen Verbindungen. Diese so genannten Raney-Katalysatoren benötigen im allgemeinen einen Aktivierungsschritt, worin das katalytisch unwirksame Metall durch Laugen entfernt wird. Im allgemeinen werden Raney-Katalysatoren als feine Pulver eingesetzt, was zwar zu einer hohen Aktivität führt, jedoch die Abtrennung aus dem Reaktionsgemisch zeitaufwändig und damit kostenintensiv macht. So werden Zucker wie z.B. Glucose technisch, heterogen mit pulverförmigen Katalysatoren zu Folgeprodukte, wie z.B. Sorbitol hydriert. Dabei wird die Hydrierung in gerührten Reaktoren absatzweise durchgeführt und der pulverförmige Katalysator muss dabei auf wändig abgetrennt werden.

Als Alternative zu pulverförmigen Raney-Katalysatoren werden Katalysatorformkörper (Tabletten, Pellets, Extrudate usw.), beispielsweise in kontinuierlich betriebenen Rieselbettreaktoren verwendet. Diese Formkörper mit einer durchschnittlichen Größe von ca. 1 bis 10 mm besitzen jedoch den Nachteil einer niedrigen Aktivität und einer unregelmäßigen Benetzung während der Reaktion. So ist die auf die Katalysatormasse normierte Geschwindigkeitskonstante der katalytischen Reaktion relativ niedrig, da die Reaktion dabei fast ausschließlich an der Tablettenoberfläche stattfindet, während der größte.Teil der Tablettenmasse im Tabletteninneren an der Reaktion nicht beteiligt ist (Diffusionslimitierung).

Der wirtschaftliche Nutzen der hochpreisigen Katalysatormetalle in tablettierten Katalysatoren ist aus diesem Grund relativ gering.

In EP-A-0094577 wird die Herstellung einer Elektrode mittels Plasmaspritzverfahren durch Auftragen einer Nickel-Unterschicht (30 bis 60 µm) auf einem Träger aus Weicheisen oder Stahl und darauf folgendes Auftragen einer 20 bis 60 µm dicken Raney-Schicht aus Nickel und Aluminium offenbart. Diese Methode beinhaltet den Nachteil einer Zweistufigkeit. Ein weiterer Nachteil besteht darin, dass anstelle eines Ni-Al-Legierungspulvers eine Mischung von Al- und Ni-Pulvern verwendet wird, wodurch die Ausbildung der benötigten Ni-AI-Phasen nicht gewährleistet ist. Auch die EP-A-0100659 beschreibt die Herstellung einer Kathode mit geringer Wasserstoff-Überspannung für die Chloralkalielektrolye. Die Kathode wird hergestellt durch Auftragen einer 1,41/.Al-Legierung einer definierten Kornfraktion mittels Plasmaspritzen auf ein elektrisch leitfähiges und poröses Metall (Fe und Legierungen). Die mit diesem. Verfahren zugänglichen Schichtdicken liegen im Bereich von 13 bis 508 µm. Die verwendete Zusammensetzung der Ni/Al-Legierung liegt mit >56 Gew.-% Nickel deutlich über den für Katalysatoren üblicherweise verwendeten Ni-Gehalten. Mit zunehmender Ni-Konzentration nimmt nämlich die Löslichkeit des Aluminiums ab, so dass es schwierig ist, das Aluminium während der Aktivierung herauszulösen. Grundsätzlich kann der Fachmann daher auch nicht erwarten, dass derartige für die Chloralkalielektrolyse konzipierte Materialien auch als Katalysatoren geeignet sind. Publikationen zur Elektrodenherstellung enthalten daher keine Hinweise zur Katalysatorherstellung und umgekehrt.

EP-A-0120122 offenbart ein Verfahren zur Hydrierung pflanzlicher Öle. Das Verfahren verwendet einen Katalysator mit Netzstruktur, bei dem auf einer Nickellegierungsschicht eine Raney-Nickel-Schicht vorliegt. Der Katalysator wird hergestellt, in dem die Oberfläche eines Netzes aus einer Nickellegierung, die einen Promotor enthält, mit Aluminium beschichtet wird, die beschichtete Netzoberfläche auf 660° bis 880°C erwärmt wird, so dass ein Teil des Aluminiums in den äußeren Bereich des Nickellegierungsnetzes eindringt, wobei eine kristalline Legierungsschicht gebildet wird, die hauptsächlich die Beta-Struktur in ihrem äußeren Bereich aufweist, und das Aluminium unter Bildung einer Raney-Metallschicht ausgelaugt wird. Dieser Katalysator weist verschiedene Nachteile auf. Besteht der Träger selbst aus Nickel oder einer Nickellegierung, bleiben große Teile des Nickels ungenutzt. Besteht der Träger nicht aus Nickel, muss das Nickel zunächst aufwändig als äußere Schicht auf den Träger aufgetragen werden. In beiden Fällen kann erst dann das Aluminium aufgetragen werden, welches in einem weiteren Schritt zur erwünschten. Legierungsschicht umgesetzt wird. Auch dabei bleiben jedoch größere Anteile des Nickels im Inneren der äußeren Schicht ungenutzt. Die EP-A-0091027 beschreibt die Anwendung dieses Katalysators zur Hydrierung von Aromaten, die EP-A-0091028 beschreibt die Anwendung dieses Katalysators zur Hydrierung von aromatischen Aminen und die EP-A-0087771 beschreibt die Anwendung dieses Katalysators zur Hydrierung von Kohlenmonooxid oder Kohlendioxid.

Die US 3637437 offenbart selbsttragende Raneysilber- oder Raneynickel-Schichtstrukturen zur Verwendung als Elektrodenmaterial. Diese können auf Nickelfolien aufgezogen sein. Die Geometrie der Strukturen wird nicht weiter erläutert. Eine Legierung eines Raneymetalls wird durch Plasmaspritzen auf ein metallisches Substrat aufgetragen, wobei die Spritzparameter so eingestellt sind, dass die Partikel nicht vollständig erschmolzen werden, um eine poröse Schicht zu erzeugen. Um die geforderte mechanische Stabilität zu erreichen sind die hergestellten Schichten mit 0,1 bis 2 mm jedoch sehr dick. Zur Herstellung wird somit eine große Menge des teuren Raneymetalls benötigt, was das Verfahren wirtschaftlich wenig attraktiv macht. Weiterhin sind die Materialien auf Grund ihrer Dicke und der speziellen, für thermische Spritzschichten typischen Struktur nur wenig flexibel. Eine Weiterverarbeitung zu gewölbten oder gerollten Packungen ist somit ohne Schädigung der Schicht nicht möglich.

Eine andere Methode zur Herstellung von geträgerten Raney-Katalysatoren wird in JP 63044944 beschrieben. Diese Methode beinhaltet das Auftragen von Al und Ni durch Plasmaspritzverfahren auf einer Zwischenschicht aus A1203 in Schichtdicken von 30 bis 40 µm. Die Haftung zwischen metallischen und oxidkeramischen Werkstoffen ist jedoch in der Regel deutlich geringer ist als bei Metall-Metall-Kontakten. Weiterhin ist das mehrstufige Verfahren sehr aufwändig. Durch die keramische Zwischenschicht wird eine deutlich geringere Flexibilität erreicht.

In der WO 01/47633 wird die Herstellung von Raney-Katalysatoren durch abwechselndes Auf tragen dünner Schichten von Nickel und Aluminium mittels Elektronenstrahlverdampfung beschrieben. Der Herstellung ist eine Temperaturbehandlung des Trägers bei 700 bis 1100°C unter einer sauerstoffhaltigen Atmosphäre vorgelagert. Die Schichtdicken liegen bei 0,01 bis 100 µm. Ein Nachteil stellt hier insbesondere die aufwändige Anfertigung der einzelnen Schichten bzw. die energieintensive Vorbehandlung des Trägers dar. Als Träger werden Folien, Gestricke oder Gewebe verwendet.

Das Auftragen des Raney-Materials mittels Verdampfung bzw. Verdüsung der flüssigen Metall-Komponenten mit einem Gas wird in WO 01/76737 beschrieben. Dabei soll eine Reaktion des Trägers mit den aufgetragenen Metallen stattfmden. Die optimale Eigenschaften der so entstandenen Zwischenschicht hängt entscheidend von der Temperatur des Trägers ab. Somit ist eine aufwändige Temperaturführung notwendig. Mit dieser Methoden sind Schichtdicken von 250 bis 550 µm zugänglich.

In JP 2002204957 wird die Herstellung eines Raney-Katalysators in folgenden Schritten beschrieben: Herstellung von Ni/Al-Pulver definierter Partikelgröße (44 µm), Dispergieren der Pulver zu einer wässrigen Ni/Al/Polyvinylalkohol-Suspension, Beschichten eines Metallträgers (Drahtgewebe) und anschließend Sintern bei 1200°C. Mit diesem Verfahren werden Schichtdicken von 5 bis 200 µm erhalten. Die Schichtherstellung erfolgt durch viele aufwändige Fertigungsschritte, resultierend in hohen Herstellkosten.

Die Erfinder stellten sich daher die technische Aufgabe, eine einfach herzustellende Alternative zu tablettierten Raney-Katalysatoren zu entwickeln, mit der kontinuierliche Hydrierverfahren kostengünstig gestaltet werden können. Gleichzeitig sollten die Katalysatoren in einer Festbettanordnung mit Pulverkatalysatoren vergleichbare auf die Katalysatormasse normierte Geschwindigkeitskonstanten erreichen. Weiterhin sollte der Katalysator insbesondere für die Hydrierung von Kohlehydraten geeignet sein.

Die Aufgabe wird erfindungsgemäß dadurch gelöst, dass das aktive Raney-Metall als dünne Schicht auf einem geeigneten Substrat vorliegt. Die Schichten werden mittels Verfahren des thermischen Spritzens sowie des Kaltgas-Spritzens erzeugt. Aus den beschichteten Substraten werden vorzugsweise Katalysatorpackungen aufgebaut, die in kontinuierlich betriebenen Hydrierreaktoren eingesetzt werden. Bedingt durch die dünne Katalysatorschicht und die große geometrische Oberfläche erreichen diese Katalysatoren in einer Festbettanordnung mit Pulverkatalysatoren vergleichbare, auf die Katalysatormasse normierte Geschwindigkeitskonstanten.

Gegenstand der Erfindung sind somit Katalysatorformkörper, erhältlich durch ein Verfahren, dass das thermische Aufspritzen mindestens eines katalytisch aktiven Metalls und mindestens eines katalytisch inaktiven Metalls auf einen Träger und das anschließende Entfernen des(r) inaktiven Metalls(e) umfasst.

Bei dem Verfahren des thermischen Aufspritzens wie es erfindungsgemäß verwendet wird, handelt es sich insbesondere um die in der DIN 32350 genannten Spritzverfahren.

In einer bevorzugten Ausführungsform wird das thermische Aufspritzen aus der Gruppe der Verfahren ausgewählt wird, die besteht aus: Flammspritzen, wie Hochgeschwindigkeitsflammspritzen; Detonationsspritzen; Plasmaspritzen, wie atmosphärisches Plasmaspritzen oder Niederdruckplasmaspritzen; Laserspritzen; Lichtbogenspritzen und Kaltgasspritzen. Besonders bevorzugt erfolgt das thermische Aufspritzen durch Hochgeschwindigkeitsflammspritzen, atmosphärisches Plasmaspritzen oder Kaltgasspritzen.

Beim thermischen Spritzen wird ein in der Regel pulver- oder drahtförmiger Spritzwerkstoff in einer Pistole durch Energiezufuhr vollständig oder teilweise aufgeschmolzen. Die Spritzpartikel werden dabei mit einem Gasstrahl hoher Geschwindigkeit auf das zu beschichtende Bauteil geschleudert. Beim Aufprall auf der zu beschichtenden Oberfläche flachen die Partikel unter Anpassung ihrer Form an die Oberfläche ab und kühlen schnell ab. Die nacheinander aufprallenden Partikel bilden so einen lamellenartigen Schichtaufbau. Die Haftung der Partikel auf dem Substrat und untereinander beruht dabei auf mechanischer Verklammerung, Adhäsion und chemischmetallurgischen Wechselwirkungen. Die verschiedenen Verfahren des thermischen Spritzens unterscheiden sich hinsichtlich der Art und Weise, wie der Spritzwerkstoff erwärmt und beschleunigt wird. Abhängig vom Spritzverfahren resultieren daraus unterschiedliche Geschwindigkeiten und Temperaturen der Spritzpartikel.

In einer besonders bevorzugten Ausführungsform wird die katalytisch aktive Schicht durch Plasmaspritzen hergestellt. Beim Plasmaspritzen werden durch Erzeugung eines ionisierten Gases (Plasma) die höchsten Pruzessiemperaturen von bis zu 25000°C erzeugt.

Die höchsten Partikelgeschwindigkeiten und damit die dichtesten und am besten haftenden Schichten lassen sich dagegen mit dem Hochgeschwindigkeits-Flammspritzen erzielen, weshalb diese Verfahren erfindungsgemäß ebenfalls bevorzugt ist. Bei diesem Verfahren wird ein Brennstoff-Sauerstoff-Gemisch in einer Brennkammer unter hohem Druck aber viel niedrigeren Temperaturen als beim Plasmaspritzen verbrannt. Die Brenngase und die darin eingebrachten Pulverpartikel werden dann in einer Düse auf sehr hohe Geschwindigkeiten beschleunigt.

Im Gegensatz zu den oben beschriebenen Verfahren sind die Prozesstemperaturen beim Kaltgasspritzen so niedrig, dass kein Aufschmelzen des Beschichtungspulvers erfolgt. Durch die Expansion eines unter sehr hohem Druck stehenden Gases (in der Regel Stickstoff oder Helium) werden die Partikel auf so hohe Geschwindigkeiten beschleunigt, dass sie durch ihre hohe kinetische Energie ohne aufzuschmelzen beim Aufprall miteinander verklammern und teilweise verschweißen. Voraussetzung dafür ist, dass der Beschichtungswerkstoff geeignete mechanische Eigenschaften aufweist. Dies gilt für die meisten metallischen Legierungen.

Erfindungsgemäß ließen sich die besten Ergebnisse durch Aufspritzen einer Legierung aus katalytisch aktivem bzw. inaktiven Metall mittels Plasmaspritzen, Hochgeschwindigkeitsflammspritzen oder Kaltgasspritzen erzielen. Es wurden flexible und dennoch stabile Schichten erhalten, die den Aufbau gewölbter Strukturen ohne Beschädigung überstanden und sich zu hochaktiven Hydrierkatalysatoren aktivieren ließen.

Bei dem katalytisch aktiven Metall handelt es sich erfmdungsgemäß um mindestens ein in einer heterogen katalysierten Reaktion einsetzbaren katalytisch aktives Metall. Dieses wird bevorzugt ausgewählt aus der Gruppe, die besteht aus: Nickel, Silber, Kupfer, Cobalt, Eisen, Ruthenium, Palladium und Platin. Diese Metalle vermögen so genannte Raney-Katalysatoren zu bilden, in dem ein mit ihnen legiertes katalytisch inaktives Metall, wie unten beschrieben, aus der Legierung herausgelöst wird.

In einer besonders bevorzugten Ausführungsform stellen Nickel oder Cobalt das katalytisch aktive Metall dar. Besonders bevorzugt ist Nickel.

In einer möglichen Ausführungsform kann zusätzlich zu dem katalytisch aktiven Metall mindestens ein Promotormetall aufgespritzt werden, das nach dem Entfernen des katalytisch inaktiven Metalls in der aufgespritzten katalytisch aktiven Schicht zumindest zum Teil in wirksamer Menge zurückbleibt.

Beispiele solcher Promotoren schließen übliche Promotoren bei Raney-Katalysatoren ein.

In einer besonders bevorzugten Ausführungsform werden das katalytisch aktive Metall und das katalytisch inaktive Metalls sowie gegebenenfalls ein von diesen Metallen verschiedenes Promotormetall in Form einer Legierung aufgespritzt. Dies hat den Vorteil, dass sich im Gegensatz zum Auftrag voneinander separierter Pulver der Metalle, intermetallische Phasen ausbilden, woraus nach dem Auslaugen sehr viel feinteiligere bzw. porösere katalytisch aktive Schichten mit großer Oberfläche resultieren.

Das Atomverhältnis des katalytisch aktiven Metalls und des katalytisch inaktiven Metalls hängt ab von der Art des katalytisch aktiven und katalytisch inaktiven Metalls und der sich daraus bildenden intermetallischen Phasen. Im allgemeinen liegt das Atomverhältnis von katalytisch aktivem zu inaktiven Metall bevorzugt bei etwa 50 : 50 bis 10 : 90, bevorzugt bei 40 : 60 bis 20 : 80. Bevorzugt liegt das Atomverhältnis von katalytisch aktivem zu inaktiven Metall von 35 : 65 bis 25 : 75. Im Falle der Verwendung von Nickel und Aluminium als eine der bevorzugten Ausführungsformen liegt das Atomverhältnis von Nickel zu Aluminium bevorzugt bei etwa 50 : 50 bis 10 : 90. Das Gewicht des katalytisch aktiven Metalls, insbesondere des Nickels bezogen auf die Gesamtmenge der aufgetragenen, noch nicht aktivierten Katalysatorschicht beträgt bevorzugt zwischen 20 und 60 Gew.-%, bevorzugter zwischen 45 und 55 Gew.-%.

Die Schichtdicke der aufgespritzten katalytisch aktiven Schicht beträgt bevorzugt weniger als 100 µm, noch bevorzugter weniger als 90 µm. Eine Schichtdicke von mehr als 100 µm führt bezogen auf die eingesetzte Katalysatormenge zu einer zu geringen katalytischen Aktivität, da dann die inneren Bereich der katalytisch aktiven Schicht diffusionsbedingt nicht mehr ausreichend erreicht werden. Des weiteren nimmt die Flexibilität der Formkörper ab, so dass diese in einigen Fällen nicht ohne Beschädigung der katalytisch aktiven Schicht in gewellte bzw. gewölbte Formkörper verarbeitet werden können

Die vorstehenden Angaben zur Schichtdicke beziehen sich sowohl auf die Schichtdicke der aufgespritzten noch nicht ausgelaugten katalytisch aktiven Schicht als auch auf die Schichtdicke der ausgelaugten Raney-Metallschicht. Mit anderen Worten unterliegt die Schichtdicke beim Auslaugen keiner substantiellen Veränderung.

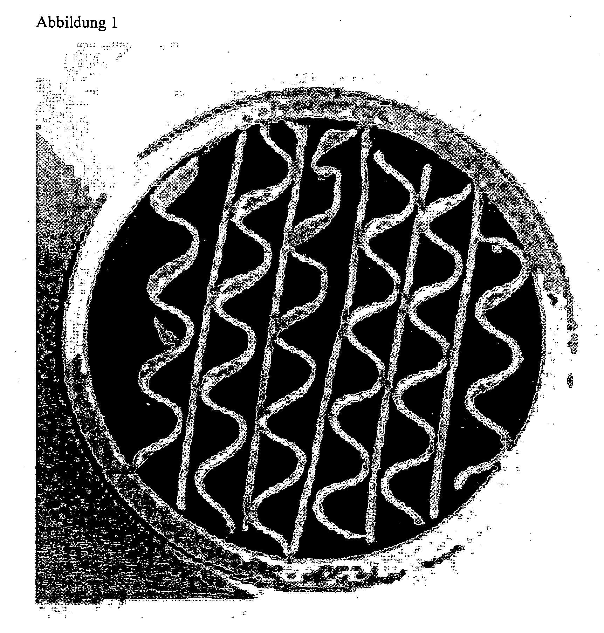

Der Begriff Formkörper im Zusammenhang mit dem erfindungsgemäßen Katalysatorformkörper meint im Rahmen der Erfindung jeden regelmäßig geformten dreidimensionalen Körper insbesondere im Unterschied zu einem unregelmäßig geformten Pulver. Bei dem Formkörper handelt es sich bevorzugt um einen schichtartigen Formkörper, der gerade und/oder gewölbt, wie zum Beispiel gewellt sein kann. Die Form des erfindungsgemäßen Formkörpers wird naturgemäß durch die Form des Trägers bestimmt. Erfindungsgemäß können zum Beispiel schichtförmige Träger von einer oder beiden Seiten durch thermisches Spritzen beschichtet werden. Die so erhaltenen schichtartigen Formkörper können so verwendet werden oder gebogen werden und in mehreren Lagen übereinander gelegt werden, um die katalytisch wirksame Oberfläche bezogen auf den Strömungsquerschnitt eines Reaktors zu erhöhen. Eine solche Ausführungsform ist beispielsweise in Abbildung 1 gezeigt. Die erfindungsgemäßen Katalysatorformkörper bestehen bevorzugt aus einem oder mehreren schichtförmigen und/oder gewölbten schichtförmigen Elementen. Mehrere erfmdungsgemäße Katalysatorformkörper können zu Katalysatorpackungen miteinander verbunden werden.

Die Ausbildung der Formkörper in eine gewünschte Endform erfolgt besonders bevorzugt vor dem Auslaugen des katalytisch inaktiven Metalls.

In einer bevorzugten Ausführungsform weisen die Träger eine kompakte, im wesentlichen keine Öffnungen aufweisende Form auf. Insbesondere ist eine poröse Struktur des Trägers weniger bevorzugt. Insbesondere handelt es sich bei dem Träger bevorzugt um dünne Schichten, wie Folien oder Bleche.

Der Träger wird bevorzugt aus einem Metall oder einer Metalllegierung ausgewählt. Besonders bevorzugte Trägermaterialien sind Stahl, rostfreier Stahl, Edelstahl, bzw. weitere ähnliche Materialien.

Ein besonders bevorzugter erfindungsgemäßer Formkörper weist einen Träger aus Edelstahl, besonders bevorzugt Edelstahl mit der Werkstoff-Nr. 1.4767 (Bezeichnung nach "Stahleisenliste", 8. Auflage, Seiten 87. 89 und 101, Hrsg. Verein Deutscher Eisenhüttenleute, Verlag Stahleisen mbH, Düsseldorf 1990) auf, und die katalytisch aktive Schicht wird durch Aufspritzen einer Nickel/Aluminumlegierung (bevorzugt im Atomverhältnis Ni/A1 von 10 : 90 bis 50 : 50, bevorzugt 40 : 60 bis 20 : 80) bevorzugt durch Plasmaspritzen oder Hochgeschwindigkeitsflammspritzen erhalten.

Die Dicke der Träger liegt bevorzugt bei 30 bis 350 µm. Kleinere Dicken führen zu einer zu geringen mechanischen Festigkeit der Katalysatorpackungen. Zu große Dicken sind aufgrund des hohen Gewichtes nachteilig.

Das katalytisch inaktive Metall, dass zur Herstellung der erfindungsgemäßen Katalysatorformkörper verwendet wird, wird bevorzugt aus der Gruppe ausgewählt, die aus Aluminium, Silizium, Magnesium und/oder Zink besteht. Besonders bevorzugt ist Aluminium.

Wie erwähnt wird das katalytisch inaktive Metall nach dem gemeinsamen Auftrag mit dem katalytisch aktiven Metall aus der resultierenden Auftragsschicht durch Behandlung mit mindestens einer wässrigen Lauge wenigstens zum Teil wieder entfernt.

Bevorzugt wird die Lauge ausgewählt aus wässrigen Lösungen von Alkali- oder Erdalkalimetallhydroxiden, wie Natronlauge oder Kalilauge.

Bevorzugt erfolgt das Auslaugen unter den folgenden Bedingungen: Besonders bevorzugt erfolgt das Auslaugen erfindungsgemäß mit einer 15 bis 30 gew.-%igen Natronlauge bei einer Temperatur von 60 bis 110°C bei Normaldruck oder gegebenenfalls oberhalb Normaldruck. Das Auslaugen kann unter relativer Bewegung der Lauge im Bezug auf den Formkörper erfolgen, wie zum Beispiel durch Bewegen des Formkörpers in der Lauge oder Umpumpen der Lauge. Nach der Zugabe der Lauge zu dem Formkörper kann noch eine Nachreaktion über einen Zeitraum von beispielsweise bis zu 120 Minuten erfolgen.

Durch dieses Verfahren bildet sich in an sich bekannter Weise eine Schicht des katalytisch aktiven Metalls mit hoher Oberfläche aus. Durch das erfmdungsgemäße Auftragsverfahren weist diese Schicht eine sehr hohe Stabilität gegenüber mechanischer Beanspruchung auf.

Bei dem erfindungsgemäßen Katalysatorformkörper kommt es unter den Bedingungen des thermischen Spritzens in der Regel im wesentlichen nicht zur Reaktion der aufgespritzten Metalle mit dem Trägermaterial unter Bildung intermetallischer Bindungen.

Durch Erhitzen des Trägermaterials könnte eine solche Reaktion bei Bedarf begünstigt werden.

Bei den erfindungsgemäßen Katalysatorformkörpern werden die genannten Metalle in der Regel direkt auf den Träger aufgespritzt ohne das eine Zwischenschicht aufgespritzt wird. Eine derartige Zwischenschicht sei sie metallischer oder keramischer Art ist erfindungsgemäß nicht bevorzugt, da es das Auftragsverfahren kompliziert und Haftungsprobleme auftreten können.

Die vorliegende Erfindung betrifft weiterhin ein Verfahren zur Herstellung des Katalysatorformkörpers, dass das thermische Aufspritzen mindestens eines katalytisch aktiven Metalls und mindestens eines katalytisch inaktiven Metalls auf einen Träger und das anschließende Entfernen des(r) inaktiven Metalls(e) umfasst.

Die Erfindung betrifft weiterhin einen Reaktionsreaktor, der mindestens einen erfindungsgemäßen Katalysatorformkörper enthält. Der Reaktor kann ein beliebiger für die Durchführung heterogen katalysierter Reaktionen geeigneter Reaktor sein, wie z.B. ein Festbettreaktor, ein Loopreaktor mit interner oder externer Rückführung.

Die Erfindung betrifft weiterhin einen Formkörper, erhältlich durch thermisches Aufspritzen einer Legierung von Nickel und Aluminium, die weniger als 56 Gew.-% Nickel enthält, auf einen Träger, worin die Dicke der aufgespritzten Schicht weniger als 0,1 mm (100 µm) beträgt. Bevorzugt erfolgt das Aufspritzen so, dass im wesentlichen keine Reaktion unter Bildung intermetallischer Bindungen zwischen den aufgespritzten Metallen und dem Träger erfolgt. Ein solcher Formkörper stellt ein Zwischenprodukt bei der Herstellung des erfindungsgemäßen Katalysatorformkörpers dar, aus dem durch Entfernen wenigstens eines Teils des Aluminiums aus der aufgespritzten Schicht der erfindungsgemäße Katalysatorformkörper gebildet wird.

Die Erfindung betrifft weiterhin die Verwendung des erfindungsgemäßen Katalysatorformkörpers als Hydrierkatalysator. Hydrierkatalysator meint insbesondere solche Katalysatoren, die geeignet sind, die Anlagerung von Wasserstoff an geeignete Verbindungen (hydrierbare Verbindungen) heterogen zu katalysieren. Derartige Verbindungen werden beispielsweise ausgewählt aus Kohlehydraten, ungesättigten Fetten, ungesättigten Kohlenwasserstoffen, wie Aromaten, aromatischen Aminen, Olefinen, Diolefinen, Alkinen, Enaminen, Nitril-Verbindungen, etc., Kohlenmonoxid, Kohlendioxid, etc.

Der erfindungsgemäßen Katalysatorformkörper eignet sich besonders zur Hydrierung von Kohlehydraten, wie z.B. Glucose, Mannose, Xylose, Galactose, Maltose, Lactose, etc. Besonders bevorzugt eignet sich der Katalysator zum Hydrieren von Glucose zu Sorbitol, da das Hydrieren im Vergleich zu den bekannten Verfahren in einfacher Weise selektiv mit hohen Ausbeuten gelingt.

Die Erfindung betrifft demnach weiterhin die Verwendung eines Formkörpers, erhältlich durch ein Verfahren, dass das thermische Aufspritzen mindestens eines katalytisch aktiven Metalls und mindestens eines katalytisch inaktiven Metalls auf einen Träger und das anschließende Entfernen des(r) inaktiven Metalls(e) umfasst, als Katalysator, insbesondere als Hydrierkatalysator, bevorzugt zum Hydrieren von Kohlehydraten, wie Glucose.

Die Erfindung betrifft weiterhin ein Verfahren zum Hydrieren von hydrierbaren Verbindungen, dass einen Formkörper, erhältlich durch ein Verfahren, dass das thermische Aufspritzen mindestens eines katalytisch aktiven Metalls und mindestens eines katalytisch inaktiven Metalls auf einen Träger und das anschließende Entfernen des(r) inaktiven Metalls(e) umfasst, verwendet. Bevorzugt dient das Verfahren zum Hydrieren von Kohlehydraten.

Die Bedingungen der Hydrierung hängen dabei insbesondere von der zu hydrierenden Verbindung ab. Bevorzugt erfolgt die Hydrierung von Kohlehydraten in Wasser bei Temperaturen von 40 bis 200°C und Wasserstoffdrücken im Bereich von 20 bis 100 bar.

Die Erfindung wird durch folgende Beispiele näher erläutert.

### Beispiele

### A) Herstellung des Katalysatorformkörpers:

Es wurden ebene und gewellte Träger aus Edelstahl mit einer Dicke von 0,1 mm beidseitig mit einer im Mittel 60 µm dicken Schicht aus einer Ni/Al-Legierung sowohl durch atmosphärisches Plasmaspritzen als auch durch Hochgeschwindigkeits-Flammspritzen beschichtet. Dazu wurden die Oberfläche der Träger vor dem Beschichten durch Strahlen mit Edelkorund der Körnung 355/250 µm bei einem Strahldruck von 1,5 bar aufgeraut. Die so behandelten Bleche wurden dann auf dem Probenhalter aufgespannt und beschichtet. Im Gegensatz zu den ebenen Trägem, bei denen die Spritzpistole senkrecht zur Oberfläche ausgerichtet wurde, betrug der Beschichtungswinkel bei den gewellten Blechen 70°, um eine über die gesamte Oberfläche eine möglichst gleichmäßige Schichtdicke zu erzeugen.

Das Plasmaspritzen wurde mit einer Pistole vom Typ F4 und folgenden Parametern durchgeführt:

| | |
|---|---|
| Argon-Fluss | 55 l/min |
| Wasserstoff-Fluss | 12 l/min |
| Strom | 500 A |
| Spannung | 80 V |
| Leistung | 40 kW |
| Abstand Pistole-Oberfläche | 140 mm |
| Pulvermenge | 40 g/min. |

Zum Hochgeschwïndigkeits-Flammspritzen wurde eine Pistole vom Typ Top Gun eingesetzt. Die Parameter wurden wie folgt eingestellt:

| | |
|---|---|
| Wasserstoff | 22,6 m³/h |
| Sauerstoff | 7,9 m³/h |
| Abstand Pistole-Oberfläche | 220 mm |
| Pulvermenge | 30 g/min. |

Die Aktivierung der Katalysatoren erfolgte mit 25 gew.-%-iger Natronlauge bei 50°C und zweistündiger Nachreaktion. Anschließend wurde mit Prozesswasser gewaschen.

### B) Hydrieren von Glukose:

Die Hydrierungen wurden bei folgenden Bedingungen geführt:
10 % Glukose in Wasser wurde bei 40 bar H2 und 120°C mit den aktivierten Formkörpern hydriert. Zum Vergleich wurden kommerziell erhältliche Katalysatoren von HC Starck eingesetzt. Ein Slurry-Versuch (Pulverkatalysator: Amperkat SK-NiMo 5546) und ein Versuch mit Tabletten (Amperkat SK-Ni 5586T) wurden in einem Rührkessel (Rührergeschwindigkeit: 1000 U/min) durchgeführt. Die Versuche mit den erfindungsgemäßen Formkörpern (beidseitig beschichtete gewellte und gerade Bleche aufgestapelt, L = 150 mm, D = 22 mm) wurden in einem Umlaufreaktor (Loop-Reaktor) durchgeführt, bei dem folgende Volumenströme eingestellt wurden: 550 L/h Gas und 55 L/h Flüssigkeit.

Der Vergleich der unterschiedlichen Katalysatoren erfolgte über die Geschwindigkeitskonstante (nach Geschwindigkeitsgesetz pseudo-erster Ordnung).

Tabelle 1 zeigt die Versuchsergebnisse.

**Tabelle 1**

| Katalysator | Geschwindigkertskonstante 1/s*kgKAT |
|---|---|
| Pulverkatalysator: Amperkat SK-NiMo 5546 | 0,03238 |
| Tablettenkatalysator: AMPERKAT SK-NI 5586T | 0,00306 |
| Erfindungsgemäße Raney-Struktur | 0,03511 |

Die Versuche zeigen, dass die erfindungsgemäßen Formkörper bezogen auf die Katalysatormenge eine sogar leicht verbesserte Aktivität aufweisen. Gleichzeitig weisen sie deutliche Vorteile in der Handhabung gegenüber Pulverkatalysatoren auf. Die erfindungsgemäßen Formkörper können in ihrer Form an bestimmte (Hydrier-)Prozesse in einfacher Weise angepasst bzw. für bestimmte Prozesse vorgefertigt werden. Sie können auch bereits in aktivierter Form, wie in der Form austauschbarer Elemente an Kunden geliefert werden, die diese transportieren, lagern und in einfacher Weise gegen verbrauchte Katalysatorelemente austauschen können. Die erfindungsgemäßen Formkörper lassen sich dabei in ihrer Größe und Form beliebig an einen gewünschten (Hydrier-)Prozess anpassen.

## Patentansprüche

1. Katalysatorformkörper, erhältlich durch ein Verfahren, dass das thermische Aufspritzen mindestens eines katalytisch aktiven Metalls und mindestens eines katalytisch inaktiven Metalls auf einen Träger und das anschließende Entfernen der(s) inaktiven Metalls(e) umfasst.

2. Katalysatorformkörper nach Anspruch 1, worin das thermische Aufspritzen aus der Gruppe der Verfahren ausgewählt wird, die besteht aus: Flammspritzen, wie Hochgeschwindigkeitsflammspritzen; Detonationsspritzen; Plasmaspritzen, wie atmosphärisches Plasmaspritzen oder Niederdruckplasmaspritzen; Laserspritzen; Lichtbogenspritzen und Kaltgasspritzen.

3. Katalysatorformkörper nach Anspruch 1 oder 2, worin eine Legierung, die mindestens ein katalytisch aktives Metall und mindestens ein katalytisch inaktives Metalls sowie gegebenenfalls ein von diesen Metallen verschiedenes Promotormetall enthält, aufgespritzt wird.

4. Katalysatorformkörper nach einem der Ansprüche 1 bis 3, worin die Schichtdicke der aufgespritzten katalytisch aktiven Schicht weniger als 100 µm beträgt.

5. Verfahren zur Herstellung des Katalysatorsformkörpers nach einem der Ansprüche 1 bis 4, dass das thermische Aufspritzen mindestens eines katalytisch aktiven Metalls und mindestens eines katalytisch inaktiven Metalls auf einen Träger und das anschließende Entfernen des(r) inaktiven Metalls(e) umfasst.

6. Reaktionsreaktor, enthaltend den Katalysatorformkörper nach einem der Ansprüche 1 bis 4.

7. Formkörper, erhältlich durch thermisches Aufspritzen einer Legierung von Nickel und Aluminium, die weniger als 56 Gew.- % Nickel enthält, auf einen Träger, worin die Dicke der aufgespritzten Schicht weniger als 0,1 mm beträgt.

8. Verwendung des Katalysatorformkörpers nach einem der Ansprüche 1 bis 4 als Hydrierkatalysator.

9. Verwendung eines Formkörpers, erhältlich durch ein Verfahren, dass das thermische Auf spritzen mindestens eines katalytisch aktiven Metalls und mindestens eines katalytisch inaktiven Metalls auf einen Träger und das anschließende Entfernen des(r) inaktiven Metalls(e) umfasst, als Katalysator.

10. Verfahren zum Hydrieren von hydrierbaren Verbindungen, dass einen Formkörper, erhältlich durch ein Verfahren, dass das thermische Aufspritzen mindestens eines katalytisch aktiven Metalls und mindestens eines katalytisch inaktiven Metalls auf einen Träger und das anschließende Entfernen des(r) inaktiven Metalls(e) umfasst, verwendet.
